(19) **Europäisches Patentamt**
**European Patent Office**
**Office européen des brevets**

(11) **EP 4 205 027 B1**

(12) **EUROPÄISCHE PATENTSCHRIFT**

(45) Veröffentlichungstag und Bekanntmachung des Hinweises auf die Patenterteilung:
**28.01.2026 Patentblatt 2026/05**

(21) Anmeldenummer: **21762594.6**

(22) Anmeldetag: **09.08.2021**

(51) Internationale Patentklassifikation (IPC):
*A61B 6/42* (2024.01)    *G01N 23/04* (2018.01)
*G01T 1/20* (2006.01)    *G06V 10/54* (2022.01)
*G06V 10/75* (2022.01)    *G06T 7/00* (2017.01)

(52) Gemeinsame Patentklassifikation (CPC):
**G06V 10/758; A61B 6/4216; G01N 23/04; G01T 1/2012; G06T 7/001; G06V 10/54; G06V 10/751; G06V 10/759;** G06T 2207/10116

(86) Internationale Anmeldenummer:
**PCT/DE2021/100678**

(87) Internationale Veröffentlichungsnummer:
**WO 2022/042794 (03.03.2022 Gazette 2022/09)**

(54) **VERFAHREN ZUR BESTIMMUNG DER SPEICHERFUNKTIONALITÄT EINER SPEICHERFOLIE FÜR RÖNTGENBILDER**

METHOD FOR DETERMINING THE STORAGE FUNCTIONALITY OF AN IMAGING PLATE FOR X-RAY IMAGES

PROCÉDÉ DE DÉTERMINATION DE LA FONCTIONNALITÉ DE STOCKAGE D'UNE PLAQUE D'IMAGERIE POUR IMAGES RADIOLOGIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.08.2020 DE 102020122196**

(43) Veröffentlichungstag der Anmeldung:
**05.07.2023 Patentblatt 2023/27**

(73) Patentinhaber: **Dürr Dental SE**
**74321 Bietigheim-Bissingen (DE)**

(72) Erfinder:
• **HACK, Alexander**
**88400 Biberach (DE)**
• **LADIKOS, Alexander**
**85221 Dachau (DE)**
• **JAGODA, Simon**
**81249 München (DE)**

• **WIECZOREK, Matthias**
**81827 München (DE)**

(74) Vertreter: **Frenkel, Matthias Alexander**
**Wuesthoff & Wuesthoff**
**Patentanwälte und Rechtsanwalt PartG mbB**
**Schweigerstraße 2**
**81541 München (DE)**

(56) Entgegenhaltungen:
**WO-A1-2019/053171**    **US-A1- 2007 156 379**

• **BERMUDEZ ARIANA ET AL: "A First Glance to the Quality Assessment of Dental Photostimulable Phosphor Plates with Deep Learning", 2020 INTERNATIONAL JOINT CONFERENCE ON NEURAL NETWORKS (IJCNN), IEEE, 19 July 2020 (2020-07-19), pages 1 - 6, XP033831116, DOI: 10.1109/ IJCNN48605.2020.9206779**

**EP 4 205 027 B1**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

HINTERGRUND DER ERFINDUNG

1. Gebiet der Erfindung

Die Erfindung betrifft ein Verfahren zur Bestimmung der Speicherfunktionalität einer Speicherfolie für Röntgenbilder.

2. Beschreibung des Standes der Technik

In der Röntgentechnik, insbesondere in der dentalmedizinischen Röntgentechnik, werden heutzutage vielfach Speicherfolien zur Aufnahme von Röntgenbildern verwendet. Diese Speicherfolien umfassen ein Phosphormaterial, das in einer transparenten Matrix eingebettet ist. Dadurch entstehen Speicherzentren, die durch einfallendes Röntgenlicht in angeregte metastabile Zustände gebracht werden. Belichtet man eine solche Speicherfolie mittels eines Röntgengeräts, enthält die Speicherfolie ein latentes Bild in Form angeregter und nicht angeregter Speicherzentren.

Zum Auslesen der Speicherfolie wird diese in einer Scanvorrichtung punktweise mit Ausleselicht abgetastet, wodurch die metastabilen Zustände der angeregten Speicherzentren unter Abgabe von Fluoreszenzlicht relaxieren. Dieses Fluoreszenzlicht kann mithilfe einer Detektoreinheit erfasst werden, so dass man mit einer entsprechenden Auswerteelektronik ein digitales Röntgenbild erhält. Für den Auslesevorgang werden beispielsweise Trommel- oder Flachbettscanner eingesetzt.

Ein großer Vorteil der Speicherfolientechnik besteht in der Wiederverwendbarkeit der Speicherfolien. Eine Speicherfolie kann nach dem Auslesen, bei dem ohnehin die in der Folie gespeicherte Bildinformation gelöscht wird, für weitere Aufnahme- und Speichervorgänge eingesetzt werden. Verschiedene Alterungsvorgänge setzen hierbei der Wiederverwendbarkeit Grenzen. Einerseits können sich im Laufe der Zeit lokale Speicherzentren ausbilden, die nicht mehr anregbar sind und somit im Röntgenbild dunkel bleiben. Andererseits können im praktischen Betrieb auch mechanische Belastungen auf der Oberfläche der Speicherfolie Kratzer oder punktförmige Beschädigungen hinterlassen.

Es ist daher wünschenswert, die Speicherfunktionalität einer Speicherfolie beurteilen zu können. Man kann hierfür beispielsweise die Speicherfolie in Augenschein nehmen und die Oberfläche nach Kratzern oder anderen Beschädigungen untersuchen und entsprechend klassifizieren. Des Weiteren kann man die Anzahl an Aufnahmen, die mit der Speicherfolie gemacht wurden, oder die Anzahl an Auslesevorgängen zählen. Als weiterer Faktor kann die Lebensdauer der Speicherfolie anhand eines Produktionsdatums in Betracht gezogen werden. Diese Maßnahmen lassen aber keine verlässliche Aussage über die tatsächliche Speicherfunktionalität der Speicherfolie zu.

Die Druckschrift WO 2019/053171 A1 beschreibt ein Verfahren zur Bestimmung der Qualität einer Speicherfolie und sieht unter anderem die Schritte des Ermitteln eines Signal-zu-Rauschen-Verhältnisses sowie das Durchführen einer Kantenerkennung vor.

Die Veröffentlichung Bermudez Ariana et al.: "A First Glance to the Quality Assessment of Dental Photostimulable Phosphor Plates with Deep Learning", 2020 INTERNATIONAL JOINT CONFERENCE ON NEURAL NETWORKS (IJCNN}, IEEE, 19. Juli 2020 (2020-07-19), Seiten 1-6, beschreibt einen Ansatz basierend auf neuronalen Netzen zur Ermittlung der Schäden einer Speicherfolie.

Die Druckschrift US 2007/156379 A1 beschreibt verschiedene Methoden und Systeme zur Nutzung von Entwurfsdaten in Kombination mit Inspektionsdaten. Eine computerimplementierte Methode zur Bestimmung einer Position von Inspektionsdaten im Entwurfsdatenraum umfasst das Ausrichten von Daten, die von einem Inspektionssystem für Ausrichtungsstellen auf einem Wafer erfasst wurden, mit Daten für vorgegebene Ausrichtungsstellen. Das Verfahren umfasst auch die Bestimmung der Positionen der Ausrichtungsstellen auf dem Wafer im Entwurfsdatenraum auf der Grundlage der Positionen der vorgegebenen Ausrichtungsstellen im Entwurfsdatenraum.

ZUSAMMENFASSUNG DER ERFINDUNG

Es ist eine Aufgabe der Erfindung, ein Verfahren zur Bestimmung der Speicherfunktionalität einer Röntgenspeicherfolie anzugeben, das die oben genannten Nachteile vermeidet und es insbesondere ermöglicht, eine genaue Aussage über die Speicherfunktionalität und -qualität der Speicherfolie zu treffen.

Die Aufgabe wird durch ein Verfahren zur Bestimmung der Speicherfunktionalität einer Röntgenfolie gemäß dem unabhängigen Anspruch gelöst. Das erfindungsgemäße Verfahren weist die Schritte auf: Bereitstellen einer Vielzahl von digitalen Röntgenbildern der Speicherfolie; Durchführen einer Kreuzkorrelations-Operation zwischen einem Röntgenbild und anderen Röntgenbildern der Speicherfolie für relevante Bildbereiche; Zusammensetzen der durch die Kreuzkorrelations-Operation gelieferten Korrelationswerte zu Merkmalsbildern; Klassifizieren der Speicherfunktionalität eines Pixels eines Merkmalsbildes anhand der Korrelationswerte anderer Pixel in der Umgebung des Pixels.

Die Erfinder haben erkannt, dass für die Bestimmung der Speicherfunktionalität und der - qualität einer Speicherfolie eine Analyse der mit derselben Speicherfolie erstellten Röntgenbilder eine gute Basis bildet. Defekte auf der Speicherfolie, die für eine Verschlechterung der Speicherfunktionalität verantwortlich sein können, sind ortsfest. Für ein Erkennen solcher ortsfesten Strukturen ist ein reines pixelbasiertes Vergleichen der einzelnen Röntgenbilder in der Regel nicht erfolgreich.

Selbst beim Auslesen derselben Speicherfolie mit derselben Auslesevorrichtung können Röntgenbilder entstehen, die sich auf der Pixelebene unterscheiden, beispielsweise durch unterschiedliche Krümmungen der Speicherfolie während des Auslesevorgangs. Zudem können die verschiedenen Röntgenbilder - beispielsweise Röntgenaufnahmen von Backenzähnen - bei einem 1-zu-1-Vergleich auf der Pixelebene in bestimmten Bereichen immer ähnliche Strukturen aufweisen. Dies würde zu falsch-positiven Ergebnissen führen, d.h. es wird ein Pixel als unveränderlich und damit als nicht mehr verwendbar erkannt, obwohl dieser Schluss darauf beruht, dass an diesem Pixel tatsächlich immer eine Struktur abgebildet ist.

Um dennoch die Speicherfunktionalität einer Röntgenspeicherfolie zu bestimmen, wird erfindungsgemäß eine Kreuzkorrelations-Operation zwischen den verschiedenen vorhandenen digitalen Röntgenbildern durchgeführt. Dabei ist es vorteilhaft, wenn die Korrelationsoperation nur auf relevanten Bildbereichen durchgeführt wird und nicht relevante Bildbereich wie beispielsweise Markierungen der Speicherfolie zur Ausrichtung oder/und der möglicherweise ebenfalls in dem Röntgenbild abgebildete Speicherfolienrand von der Operation ausgeschlossen werden.

Die Kreuzkorrelationsoperation liefert Korrelationswerte, die sich wiederum zu einem Bild zusammensetzen lassen. Das aus Korrelationswerten bestehende Bild wird Merkmalsbild genannt, da es aufgrund der Korrelationsoperation Merkmale der zugrundeliegenden Speicherfolie erkennen lässt. Es bilden sich auf diese Weise vor allem solche Merkmale ab, die auf beiden betrachteten Röntgenbildern gleich oder sehr ähnlich sind und damit vermutlich Merkmale der Speicherfolie und keine abgebildeten Strukturen sind.

Ein Pixel eines solchen Merkmalsbildes kann nun hinsichtlich seiner Speicherfunktionalität oder -fähigkeit klassifiziert werden. Hierzu werden die Korrelationswerte derjenigen Pixel betrachtet, die sich in seiner Umgebung befinden. Anhand der Höhe des Korrelationsgrades der umgebenden Pixel kann bestimmt werden, ob der betrachtete Pixel hoch korreliert ist. Ist dies der Fall, wird seine Speicherfunktionalität als fehlerhaft klassifiziert.

Bei einer bevorzugten Ausführungsform des Verfahrens werden bei dem Schritt des Klassifizierens nur Pixel in der Umgebung herangezogen, deren Korrelationswert einen Korrelationsschwellenwert übersteigt. Es wird also ein Korrelationsschwellenwert definiert, mit dem die Pixel in der Umgebung verglichen werden. Bevorzugt werden dann nur diejenigen Pixel für das Klassifizieren herangezogen, deren Korrelationswert diesen Korrelationsschwellenwert steigt.

Alternativ oder zusätzlich kann ein mittlerer Korrelationswert für die Pixel der Umgebung errechnet werden. Dieser mittlere Korrelationswert kann ebenfalls mit einem Korrelationsschwellenwert verglichen werden.

Bevorzugt wird die Kreuzkorrelations-Operation paarweise zwischen einem Röntgenbild und den übrigen bereitgestellten Röntgenbildern derselben Speicherfolie durchgeführt. So kann auf einer Anzahl n bereitgestellter Röntgenbilder die Kreuzkorrelations-Operation ½ n (n-1)-mal durchgeführt werden. Es ergeben sich ½ n (n-1) Merkmalsbilder. Bevorzugt findet diese Kreuzkorrelations-Operation auf mindestens 10, bevorzugt auf mindestens 20, besonders bevorzugt auf mindestens 30 Röntgenbildern statt.

Bei der Kreuzkorrelations-Operation kann die Berechnung der Korrelation beispielsweise auf einem kleinen Ausschnitt des Gesamtbildes erfolgen. Dieser Ausschnitt, hier als Korrelationsfeld bezeichnet, wird so klein gewählt, dass eine zuverlässige Erkennung von typischen Beschädigungen wie Kratzern erfolgen kann. Bei einem typischen Verhältnis von 25 s/Speicherzentrum bzw. je Pixel ist eine Seitenlänge von 400 $\mu$m oder 16 Pixeln vorteilhaft.

Bevorzugt wird dieses Korrelationsfeld für die Berechnung der Kreuzkorrelation Pixel für Pixel verschoben und jedes Mal eine Korrelationsberechnung durchgeführt.

Zur Verbesserung des Verfahrens kann bevorzugterweise eine Ausrichtungserkennung oder/und eine Kantenerkennung an den digitalen Röntgenbildern vor dem Durchführen der Kreuzkorrelations-Operation durchgeführt werden. Auf diese Weise verbessert sich der Kontrast der Merkmalsbilder.

Besonders bevorzugt weisen die digitalen Röntgenbilder Nutzdaten auf. Es ist also nicht notwendig, zur Feststellung der Speicherfunktionalität einer Röntgenspeicherfolie an dieser Leerbelichtungen oder Ähnliches durchzuführen. Vielmehr kann im laufenden Betrieb anhand der aufgezeichneten digitalen Röntgenbilder derselben Speicherfolie deren Speicherfunktionalität sozusagen im Hintergrund überwacht und analysiert werden. Es ist also weder notwendig, bestimmte Aufnahmeparameter zu verwenden noch besondere für den Zweck der Bestimmung der Speicherfunktionalität gedachte Röntgenbilder mit der Speicherfolie aufzunehmen. Es muss lediglich der mit derselben Speicherfolie erzeugte gesamte Röntgenbilder-Pool oder Teile daraus, beispielsweise die letzten 30 Aufnahmen oder die Aufnahmen der letzten X Tage, für die Analyse bereitgestellt werden.

Konkret handelt es sich bevorzugt bei der Kreuzkorrelations-Operation um eine lokal normalisierte Berechnung, beispielsweise mit dem LNCC-Algorithmus (LNCC = Locally Normalized Cross Correlation).

Bevorzugt handelt es sich bei der Umgebung des Pixels, die für die Klassifizierung in Betracht gezogen wird, um ein Quadrat mit einer Seitenlänge zwischen 10 $\mu$m und 1000 $\mu$m, bevorzugt zwischen 200 $\mu$m und 500 $\mu$m, besonders bevorzugt von 400 $\mu$m.

Bei einer Weiterbildung des Verfahrens wird die Speicherfunktionalität des Pixels als fehlerhaft klassifiziert, wenn die Anzahl der Pixel in der Umgebung, deren Korrelationswert einen Korrelationsschwellenwert übersteigt, größer oder gleich einer maximalen Anzahl ist. Bevorzugt liegt diese maximale Anzahl bei 25 % aller Pixel in dem Analysefeld. Wenn also bei 25 % aller Pixel in der Umgebung des betrachteten Pixels der Korrelationswert über einem Korrelationsschwellenwert liegt, wird die Speicherfunktionalität dieses Pixels als fehlerhaft klassifiziert.

Die relevanten Bildbereiche, auf denen die Kreuzkorrelations-Operation durchgeführt wird, schließen Markierungen der Speicherfolie wie beispielsweise Ausrichtungsmarkierungen oder/und den Rand der Speicherfolie, der eventuell auf dem Röntgenbild erkennbar ist, aus.

Zusätzlich kann bei dem Klassifizieren der Speicherfähigkeit eines Pixels das Aufnahmedatum eines Röntgenbildes bei dem Klassifizierungsschritt berücksichtigt werden.

Die Aufgabe wird außerdem durch eine Vorrichtung zur Bestimmung der Speicherfähigkeit einer Speicherfolie für Röntgenbilder gelöst. Die erfindungsgemäße Vorrichtung weist ein Speicherfolienauslesegerät zum Auslesen einer belichteten Speicherfolie auf sowie eine Steuereinrichtung, die dazu eingerichtet ist, ein Verfahren wie vorstehend beschrieben auszuführen.

KURZE BESCHREIBUNG DER ZEICHNUNGEN

Nachfolgend werden Ausführungsbeispiele der Erfindung anhand der Zeichnungen näher erläutert. In diesen zeigen:

Figur 1    eine erste Ausführungsform;

Figur 2    das Erstellen von geeigneten Röntgenbildern;

Figur 3    eine zweite Ausführungsform;

Figur 4    eine Veranschaulichung eines ersten Teils der zweiten Ausführungsform, und

Figur 5    eine Veranschaulichung eines zweiten Teils der zweiten Ausführungsform.

BESCHREIBUNG BEVORZUGTER AUSFÜHRUNGSBEISPIELE

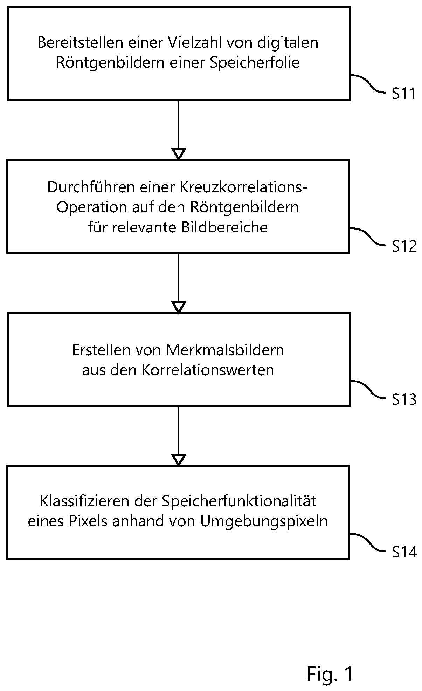

Figur 1 veranschaulicht das erfindungsgemäße Verfahren in einer ersten Ausführungsform.

In einem ersten Schritt (S11) wird eine Vielzahl digitaler Röntgenbilder einer Speicherfolie bereitgestellt. Die Röntgenbilder stammen von derselben Speicherfolie. Damit ist gemeint, dass jedes der Röntgenbilder von der physikalisch identischen Speicherfolie stammt. Auf diese Weise ist es möglich, das eventuelle Vorhandensein von Defekten auf oder in der Folie zu erkennen. Bei der Vielzahl an Röntgenbildern kann es sich beispielsweise um die letzten 10, 20 oder 30 Aufnahmen mit tatsächlichen Nutzdaten handeln. Es kann aber prinzipiell jegliche beliebige Untermenge aus den vorhandenen Röntgenbildern gebildet werden. Die Nutzdaten können beispielsweise aus dem Verwenden der Speicherfolie für normale diagnostische Zwecke stammen. Der Schritt des Bereitstellens kann beispielsweise umfassen, anhand einer eindeutigen Kennung, welche der Speicherfolie zugewiesen ist, die zu dieser Speicherfolie gehörigen Röntgenbilder aus einer größeren Anzahl an Röntgenbilder herauszufiltern. Die Speicherfolie kann beispielsweise einen RFID-Tag aufweisen, auf dem eine eindeutige Identifikationsnummer abgespeichert ist. Beim Auslesen der Speicherfolie mittels des Speicherfolienauslesegeräts kann diese Identifikationsnummer abgefragt und dem Röntgenbild - beispielsweise in den Metadaten - hinzugefügt und mit diesem abgespeichert werden.

Zusätzlich oder alternativ kann der Schritt des Bereitstellens ein Filter nach dem Aufnahmedatum der Röntgenbilder derselben Speicherfolie umfassen.

In einem weiteren Schritt (S12) wird ein Kreuzkorrelations-Algorithmus auf die bereitgestellten Röntgenbilder für relevante Bildbereiche angewendet. Die relevanten Bildbereiche können bereits vor dem Anwenden des Kreuzkorrelationsalgorithmus aus den bereitgestellten Röntgenbildern ausgewählt worden sein. Bei dem Anwenden des Kreuzkorrelationsalgorithmus werden paarweise Röntgenbilder hinsichtlich ihrer Korrelation überprüft.

Die aus dem Kreuzkorrelationsalgorithmus erhaltenen Korrelationswerten werden in einem weiteren Schritt (S13) zu einem Merkmalsbild zusammengesetzt. In diesem Merkmalsbild sind Merkmale erkennbar, die nicht aus den Nutzdaten stammen, sondern welche die Speicherfunktionalität der Speicherfolie wiedergeben.

Um einen Pixel oder ganze Bildbereiche hinsichtlich ihrer Speicherfunktionalität zu charakterisieren, wird in einem weiteren Schritt (S14) die Umgebung eines Pixels des Röntgenbildes betrachtet. Dabei kann anhand der Korrelationswerte der Pixel, welche den untersuchten Pixel umgeben, auf die Speicherfunktionalität des untersuchten Pixels rückgeschlossen werden und dessen Speicherfunktionalität charakterisiert bzw. klassifiziert werden. Übersteigen beispielsweise die einzelnen oder alle Korrelationswerte einen bestimmten Schwellenwert, kann die Speicherfunktionalität des untersuchten Pixels entsprechend als "nicht vorhanden" oder "fehlerhaft" klassifiziert werden. Alternativ oder zusätzlich kann eine Anzahl an Pixeln, welche den Korrelationsschwellenwert erreichen oder übersteigen, in Betracht gezogen werden. Übersteigt beispielsweise ein bestimmter Prozentsatz der umgebenden Pixel einen bestimmten Schwellenwert, kann die Speicherfunktionalität des untersuchten Pixels als "nicht vorhanden" oder "fehlerhaft" gekennzeichnet werden.

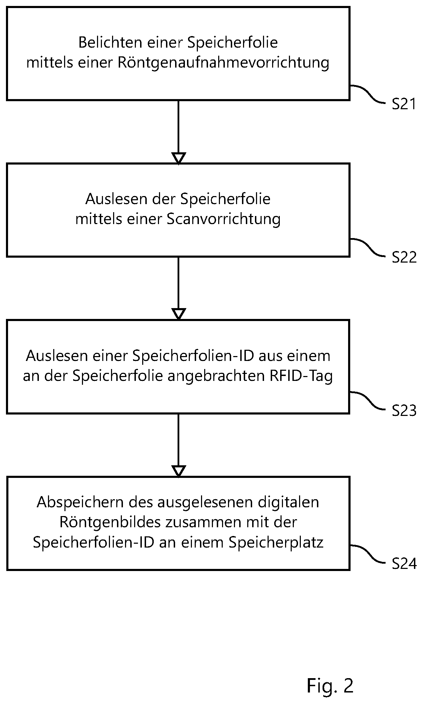

Figur 2 veranschaulicht den Vorgang des Erstellens der Röntgenbilder, die für das Untersuchen der Speicherfunktionalität einer Speicherfolie benötigt werden.

In einem ersten Schritt (S21) wird eine Speicherfolie mittels einer Röntgenaufnahmevorrichtung belichtet. Dabei handelt es sich um einen üblichen Vorgang, der keiner weiteren Erläuterung bedarf.

In einem weiteren Schritt (S22) wird die Speicherfolie mittels einer Scanvorrichtung ausgelesen. Dabei entsteht ein digitales Röntgenbild. Bei dem Auslesevorgang werden wie eingehend beschrieben die metastabile Zustände der Speicherzentren mittels eines Ausleselichts angeregt und es erfolgt die Relaxation unter Abgabe von Fluoreszenzlichts.

Gleichzeitig, davor oder danach wird ein an der Speicherfolie angebrachter RFID-Tag ausgelesen (S23), um eine eindeutig der Speicherfolie zuzuordnende Kennung zu erhalten.

Das digitale Röntgenbild wird anschließend (S24) mit der eindeutigen Kennung der Speicherfolie verknüpft und an einem Speicherplatz abgelegt. Dabei kann die Ablage der Röntgenbilder bereits nach Speicherfolie sortiert erfolgen. Alternativ kann auch bereits eine Vorsortierung der Röntgenbilder nach dem jeweiligen Aufnahmedatum erfolgen.

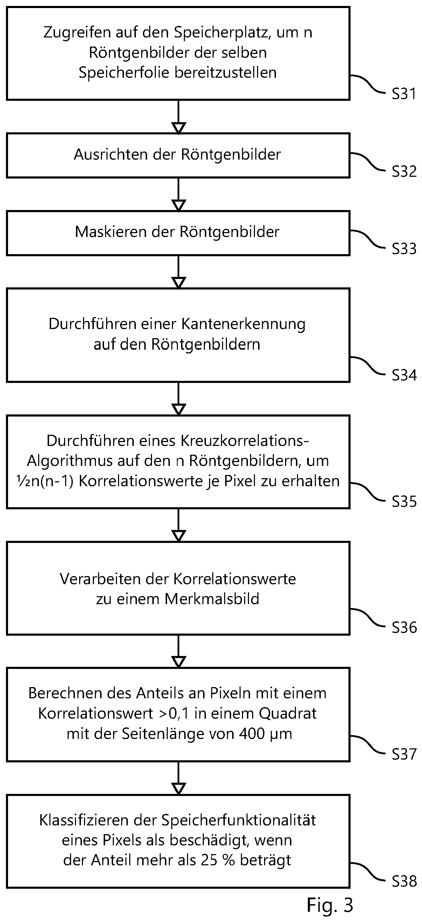

Figur 3 veranschaulicht eine zweite Ausführungsform des erfindungsgemäßen Verfahrens.

In einem ersten Schritt (S31) wird auf einen Speicherplatz zugegriffen, an dem Röntgenbilder hinterlegt sind, um n Röntgenbilder derselben Speicherfolie bereitzustellen. Das Auswählen der Röntgenbilder derselben Speicherfolie geschieht anhand einer Kennung, mit der die Röntgenbilder verknüpft sind. Beispielsweise kann die eindeutig der Speicherfolie zuordenbare Kennung in der Röntgenbild-Datei - beispielsweise den Metadaten - eingebettet sein. Die Auswahl der n Röntgenbilder umfasst in der Regel eine bestimmte Anzahl - beispielsweise 10, 20 oder 30 - der zuletzt mit derselben Speicherfolie gemachten Röntgenaufnahmen.

In einem weiteren Schritt (S32) werden die Röntgenbilder ausgerichtet. Dazu können beispielsweise in dem Röntgenbild erkennbare Ausrichtungsmarkierungen für eine erste grobe Ausrichtung und die Ränder der in dem Röntgenbild erkennbaren Röntgenfolie für eine Feinausrichtung verwendet werden.

Um den Bildinhalt der Röntgenbilder für eine Analyse besser aufzubereiten, werden in einem weiteren Schritt (S33) für die Analyse nicht verwendbare Bildinhalte maskiert. Dabei kann es sich beispielsweise um die genannten Ausrichtungsmarkierungen, die Ränder der Speicherfolie oder andere fest mit der Speicherfolie verknüpfte Bildinhalte handeln. Die weitere Analyse findet dann auf relevanten Bildinhalten unter Ausschluss der maskierten Bildinhalte statt.

Auf den maskierten Röntgenbildern wird in einem weiteren Schritt (S34) eine Kantenerkennung durchgeführt, um das Signal-zu-Rauschen-Verhältnis der Bildinhalte zu verbessern.

Auf den so aufbereiteten Röntgenbildern wird nun die Kreuzkorrelations-Operation durchgeführt (S35).

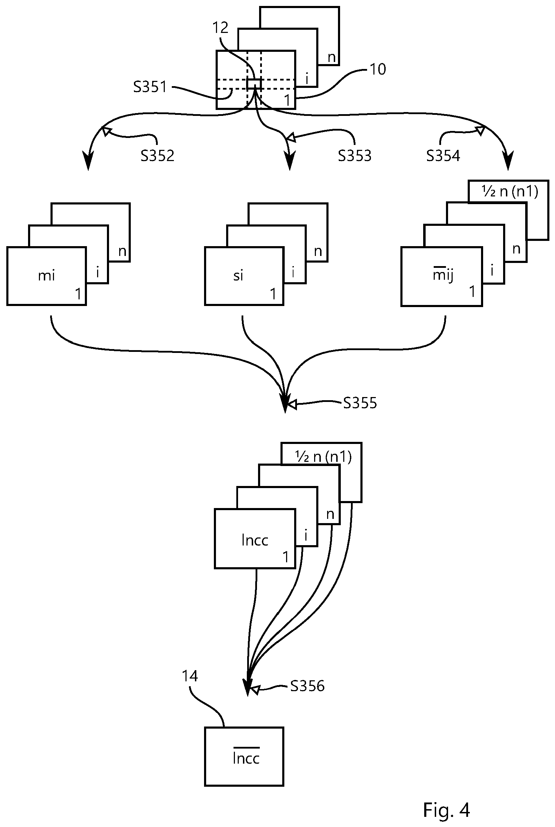

Dies ist zusätzlich in Figur 4 dargestellt.

Dabei werden zunächst für jedes der n Röntgenbilder 10 ein Mittelwert mi und eine Standardabweichung si innerhalb eines Feldes 12 berechnet. Das Feld 12, hier als Korrelationsfeld 12 definiert, umfasst einen Teil des Röntgenbildes 10, der kleiner ist als das Röntgenbild selbst. Bevorzugt ist die Größe des Korrelationsfelds 12 an die Abmessungen einer möglichen Beschädigung der Speicherfolie angepasst. Dies soll bedeuten, dass das Korrelationsfeld 12 so klein sein muss, dass eine mögliche Beschädigung noch erkennbar bleibt. Gleichzeitig sollte das Korrelationsfeld 12 so groß sein, dass eine Mittelung über eine bestimmte Anzahl an Speicherzentren bzw. Pixel erfolgt. Beispielsweise können die Abmessungen für ein solches Korrelationsfeld 100 μm x 100 μm, also beispielsweise 4 x 4 Pixel betragen.

Das Korrelationsfeld 12 wird über das gesamte Röntgenbild pixelweise verschoben (S351) und es wird für jede Position des Korrelationsfeldes 12 der Mittelwert mi (S352) sowie die Standardabweichung si (S353) berechnet. Aus dieser Berechnung entstehen Werte-Matrizen, die in etwa die Größe des ursprünglichen Bildes aufweisen. Während im Mittelbereich des Bildes jedem Pixel eine Feldposition entspricht, fallen an den Rändern des Bildes aufgrund der eigenen Größe des Korrelationsfeldes einige Pixel-Feldposition-Entsprechungen weg.

Gleichzeitig wird für jede Position des Korrelationsfeldes innerhalb des Korrelationsfeldes ein Produkt mij zwischen jedem Pixel innerhalb des Feldes und einem Pixel an gleicher Position in einem anderen Bild berechnet und über das Korrelationsfeld gemittelt (S354). Diese Operation wird für alle möglichen Bildkombinationen durchgeführt. Die so entstehenden Werte-matrizen besitzt besitzen die gleiche Größe wie diejenigen für Mittelwerte und Standardabweichung. Während allerdings die Anzahl an Matrizen für die Mittelwerte und Standardabweichungen der Anzahl an Bildern entspricht, ergibt die Produktbildung für alle möglichen Kombinationen ½ n (n-1) Wertematrizen.

Mithilfe der so gewonnenen Wertematrizen kann die lokale normierte Kreuzkorrelation für jede der ½ n (n-1) Kombinationen berechnet (S355) und über alle diese Kombinationen gemittelt (S356) werden. Die Kreuzkorrelation berechnet sich dabei wie folgt:

$$lncc = \frac{mij - mi \cdot mj}{si \cdot sj}$$

Dabei sind mi, mj der über das Korrelationsfeld gemittelte Mittelwert des Bildes i bzw. j und si, sj die zugehörigen Standardabweichungenn. mij ist das wie oben beschriebene über das Korrelationsfeld gemittelte Produkt zwischen den Bildern i und j.

Die so erhaltenen lncc-Werte für alle möglichen Bildkombinationen werden gemittelt, so dass dann eine einzige Wertematrix 14 mit den berechneten Korrelationswerten übrigbleibt (S356). Alternativ kann statt der arithmetischen Mittelwertbildung auch der Median und gegebenenfalls zusätzlich die Standardabweichung berechnet werden.

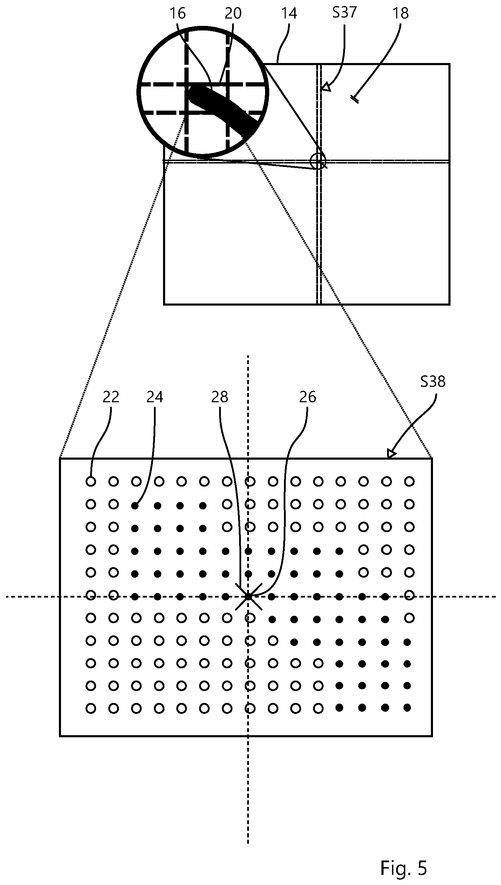

Diese Wertematrix 14 kann man als Bild darstellen (S36). In diesem Merkmals- oder Featurebild genannten Bild sind Merkmale 16, 18 (siehe Figur 5) erkennbar, die über die verschiedenen Aufnahmen, die mit derselben Speicherfolie getätigt wurden, persistieren und somit ein Merkmal der Speicherfolie und nicht der eigentlich auf den Röntgenbildern 10 abgebildeten Strukturen sind. Die eigentlichen abgebildeten Strukturen hingegen unterscheiden sich zumindest leicht von Röntgenbild zu Röntgenbild.

Um nun die Speicherfähigkeit der Speicherfolie anhand der Korrelationswerte zu charakterisieren und für bestimmte Bereiche die Speicherfähigkeit als "nicht vorhanden" oder als "fehlerhaft" zu klassifizieren, werden die Pixel des Merkmalsbildes untersucht (S37). Dies ist auch in Figur 5 veranschaulicht.

In dem Merkmalsbild 14 werden aus allen Pixeln 22 diejenigen Pixel 24 bestimmt, deren Korrelationswert innerhalb eines festgelegten Analysefeldes 20 über einem Schwellenwert liegt. Der Schwellenwert bewegt sich bevorzugt zwischen 0,07 und 0,15 und kann beispielsweise bei 0,1 liegen. Das Analysefeld 20 weist beispielhaft eine physikalische Dimension von beispielsweise 400 $\mu$m x 400 $\mu$m auf und kann - wie bereits erwähnt - an die physikalischen Dimensionen der Speicherfolie und der Art und Größe der zu erwarteten Defekte angepasst werden.

Liegt innerhalb des Analysefeldes 20 die Anzahl an Bildpunkten 22, deren gemittelter Korrelationswert auf oder über diesem Schwellenwert liegt, über einer maximalen Anzahl (S38), so wird der dem Analysefeld 20 zugehörige Bildpunkt 26 als nicht mehr funktionsfähig hinsichtlich seiner Speicherfähigkeit gekennzeichnet 28. Mit dem Analysefeld 20 wird das Merkmalsbild 14 Punkt für Punkt abgerastert, so dass am Ende diejenigen Bildstellen 28 markiert sind, deren Funktionsfähigkeit so beeinträchtigt ist, dass sie als nicht mehr funktionsfähig zu kennzeichnen sind. Als maximale Anzahl an auf oder über dem Schwellenwert liegenden Korrelationswerten kann beispielsweise eine relative Größe wie 25 % aller in dem Analysefeld 20 liegenden Korrelationswerte gewählt werden.

**Patentansprüche**

1. Verfahren zur Bestimmung der Speicherfunktionalität einer Röntgenspeicherfolie, mit den Schritten:

   a) Bereitstellen einer Vielzahl von digitalen Röntgenbildern der Speicherfolie (S11);
   b) Durchführen einer Kreuzkorrelations-Operation zwischen einem Röntgenbild und anderen Röntgenbildern der Speicherfolie für relevante Bildbereiche (S12);
   c) Zusammensetzen der durch die Kreuzkorrelations-Operation gelieferten Korrelationswerte zu Merkmalsbildern (S13);
   d) Klassifizieren der Speicherfunktionalität eines Pixels eines Merkmalsbildes anhand der Korrelationswerte anderer Pixel in der Umgebung des Pixels (S14).

2. Verfahren nach Anspruch 1, wobei bei dem Schritt des Klassifizierens nur Pixel in der Umgebung herangezogen werden, deren Korrelationswert einen Korrelationsschwellenwert übersteigt (S37).

3. Verfahren nach einem der vorhergehenden Ansprüche, mit dem Schritt: Durchführen der Kreuzkorrelations-Operation paarweise zwischen einem Röntgenbild (10) und den übrigen Röntgenbildern (10) der gleichen Speicherfolie, bevorzugt mit mindestens 10 vorherigen Röntgenbildern (10), besonders bevorzugt mit mindestens 20 vorherigen Röntgenbildern (10), insbesondere mit mindestens 30 vorherigen Röntgenbildern (10).

4. Verfahren nach einem der vorhergehenden Ansprüche, mit dem Schritt: Durchführen einer Ausrichtungserkennung oder/und einer Kantenerkennung (S34) an einem digitalen Röntgenbild vor dem Durchführen der Kreuzkorrelations-Operation.

5. Verfahren nach einem der vorhergehenden Ansprüche, wobei die digitalen Röntgenbilder Nutzdaten aufweisen.

6. Verfahren nach einem der vorhergehenden Ansprüche, wobei die Kreuzkorrelations-Operation lokal normalisiert ist.

7. Verfahren nach einem der vorhergehenden Ansprüche, wobei die Umgebung des Pixels ein Quadrat mit einer Seitenlänge zwischen 10 $\mu$m und 1000 $\mu$m, bevorzugt zwischen 200 $\mu$m und 500 $\mu$m, besonders bevorzugt von 400 $\mu$m ist.

8. Verfahren nach einem der vorhergehenden Ansprüche, wobei die Speicherfunktionalität des Pixels als fehlerhaft klassifiziert wird, wenn die Anzahl der Pixel in der Umgebung, deren Korrelationswert den Korrelationsschwellenwert übersteigt, einen Schwellenwert übersteigt (S38).

9. Verfahren nach Anspruch 8, wobei der Schwellenwert 25 % beträgt.

10. Verfahren nach einem der vorhergehenden Ansprüche, wobei die relevanten Bildbereiche des Röntgenbildes Ausrichtungsmarkierungen oder/und den Rand der Speicherfolie ausschließen (S33).

**11.** Verfahren nach einem der vorhergehenden Ansprüche, wobei das Aufnahmedatum eines Röntgenbildes bei dem Klassifizierungsschritt berücksichtigt wird.

**12.** Vorrichtung zur Bestimmung der Speicherfähigkeit einer Speicherfolie für Röntgenbilder, mit

a) einem Speicherfolienauslesegerät zum Auslesen einer belichteten Speicherfolie und
b) einer Steuereinrichtung, die dazu eingerichtet ist, ein Verfahren nach einem der vorhergehenden Ansprüche auszuführen.

**Claims**

**1.** A method for determination of the storage functionality of an X-ray imaging plate, comprising the steps of:

a) providing a plurality of digital X-ray images of the imaging plate (S11);
b) performing a cross-correlation operation between an X-ray image and remaining X-ray images of the imaging plate for relevant image regions (S12);
c) creating feature images from the correlation values obtained by the cross-correlation operation (S13); and
d) classifying the storage functionality of a pixel of a feature image based on the correlation values of other pixels in the vicinity of the pixel (S14).

**2.** The method according to claim 1, wherein in the step of classifying, only pixels in the vicinity whose correlation value exceeds a correlation threshold are used (S37).

**3.** The method according to any one of the previous claims, comprising the step of:
performing the cross-correlation operation pairwise between an X-ray image (10) and the remaining X-ray images (10) of the same imaging plate, preferably with at least 10 previous X-ray images (10), particularly preferable with at least 20 previous X-ray images (10), particularly with at least 30 previous X-ray images (10).

**4.** The method according to any one of the previous claims, further comprising the step of: performing an alignment detection or/and an edge detection (54) on a digital X-ray image before performing the cross-correlation operation.

**5.** The method according to any one of the previous claims, wherein the digital X-ray images comprise usage data.

**6.** The method according to any one of the previous claims, wherein the cross-correlation operation is locally normalized.

**7.** The method according to any one of the previous claims, wherein the vicinity of the pixel is a square with a side length between 10 $\mu$m and 1000 $\mu$m, preferably between 200 $\mu$m and 500 $\mu$m, particularly preferably of 400 $\mu$m.

**8.** The method according to any one of the preceding claims, wherein the storage functionality of the pixel is classified as faulty if the number of pixels in the vicinity whose correlation value exceeds the correlation threshold exceeds a threshold.

**9.** The method according to claim 8, wherein the threshold is 25%.

**10.** The method according to any one of the previous claims, wherein the relevant image regions of the X-ray image exclude alignment marks or/and the edge of the imaging plate (S33).

**11.** The method according to any one of the previous claims, wherein the acquisition date of the X-ray image is taken into account in the classification step.

**12.** A device for determining the storage capacity of an imaging plate for X-ray images, comprising:

a) an imaging plate readout apparatus for reading out an exposed imaging plate; and
b) a control device configured to perform a method according to any one of the previous claims.

**Revendications**

**1.** Procédé pour déterminer la fonctionnalité de stockage d'une plaque d'imagerie radiographique, comprenant les étapes consistant à :

a) fournir une pluralité d'images radiographiques numériques de la plaque d'imagerie (S11) ;
b) effectuer une opération de corrélation croisée entre une image radiographique et d'autres images radiographiques de la plaque d'imagerie pour des zones d'image pertinentes (S12) ;
c) assembler les valeurs de corrélation fournies par l'opération de corrélation croisée pour former des images de caractéristiques (S13) ;
d) classer la fonctionnalité de stockage d'un pixel d'une image de caractéristiques à l'aide des valeurs de corrélation d'autres pixels dans l'environnement du pixel (S14).

2. Procédé selon la revendication 1, dans lequel, lors de l'étape de classement, seuls sont pris en compte les pixels de l'environnement dont la valeur de corrélation dépasse une valeur seuil de corrélation (S37).

3. Procédé selon l'une des revendications précédentes, comprenant l'étape consistant à :
effectuer l'opération de corrélation croisée par paires entre une image radiographique (10) et les autres images radiographiques (10) de la même plaque d'imagerie, de préférence avec au moins 10 images radiographiques précédentes (10), de manière particulièrement préférée avec au moins 20 images radiographiques précédentes (10), en particulier avec au moins images radiographiques précédentes (10).

4. Procédé selon l'une des revendications précédentes, comprenant l'étape consistant à :
effectuer une détection d'orientation et/ou une détection de bord (S34) sur une image radiographique numérique avant d'effectuer l'opération de corrélation croisée.

5. Procédé selon l'une des revendications précédentes, dans lequel les images radiographiques numériques comprennent des données utiles.

6. Procédé selon l'une des revendications précédentes, dans lequel l'opération de corrélation croisée est normalisée localement.

7. Procédé selon l'une des revendications précédentes, dans lequel l'environnement du pixel est un carré dont la longueur des côtés est comprise entre 10 μm et 1000 μm, de préférence entre 200 μm et 500 μm, de manière particulièrement préférée égale à 400 μm.

8. Procédé selon l'une des revendications précédentes, dans lequel la fonctionnalité de stockage du pixel est classée comme défectueuse lorsque le nombre de pixels dans l'environnement, dont la valeur de corrélation dépasse la valeur seuil de corrélation, dépasse une valeur seuil (S38).

9. Procédé selon la revendication 8, dans lequel la valeur seuil est de 25 %.

10. Procédé selon l'une des revendications précédentes, dans lequel les zones d'image pertinentes de l'image radiographique excluent les repères d'alignement et/ou le bord de la plaque d'imagerie (S33).

11. Procédé selon l'une des revendications précédentes, dans lequel la date de prise de vue d'une image radiographique est prise en compte lors de l'étape de classement.

12. Dispositif pour déterminer la capacité de stockage d'une plaque d'imagerie pour images radiographiques, comprenant :

a) un lecteur de plaque d'imagerie pour lire une plaque d'imagerie exposée et
b) un dispositif de commande qui est conçu pour exécuter un procédé selon l'une des revendications précédentes.

```
┌─────────────────────────────────────┐
│  Bereitstellen einer Vielzahl von    │
│   digitalen Röntgenbildern einer     │
│          Speicherfolie               │──── S11
└─────────────────────────────────────┘
                   │
                   ▽
┌─────────────────────────────────────┐
│   Durchführen einer Kreuzkorrelations-│
│    Operation auf den Röntgenbildern  │
│       für relevante Bildbereiche     │──── S12
└─────────────────────────────────────┘
                   │
                   ▽
┌─────────────────────────────────────┐
│      Erstellen von Merkmalsbildern   │
│       aus den Korrelationswerten     │──── S13
└─────────────────────────────────────┘
                   │
                   ▽
┌─────────────────────────────────────┐
│  Klassifizieren der Speicherfunktionalität│
│  eines Pixels anhand von Umgebungspixeln│──── S14
└─────────────────────────────────────┘
```

Fig. 1

Belichten einer Speicherfolie
mittels einer Röntgenaufnahmevorrichtung

S21

Auslesen der Speicherfolie
mittels einer Scanvorrichtung

S22

Auslesen einer Speicherfolien-ID aus einem
an der Speicherfolie angebrachten RFID-Tag

S23

Abspeichern des ausgelesenen digitalen
Röntgenbildes zusammen mit der
Speicherfolien-ID an einem Speicherplatz

S24

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2019053171 A1
- US 2007156379 A1

**In der Beschreibung aufgeführte Nicht-Patentliteratur**

- A First Glance to the Quality Assessment of Dental Photostimulable Phosphor Plates with Deep Learning. **BERMUDEZ ARIANA et al.** 2020 INTERNATIONAL JOINT CONFERENCE ON NEURAL NETWORKS (IJCNN. IEEE, 19 July 2020, 1-6